# EUROPÄISCHE PATENTSCHRIFT

(11) **EP 0 461 474 B1**
(45) Veröffentlichungstag und Bekanntmachung des Hinweises auf die Patenterteilung: **20.07.1994**
(21) Anmeldenummer: 91108769.0
(22) Anmeldetag: 29.05.1991
(51) Int. Cl.: A61M 29/02

(54) **Ballonkatheter mit Hülse**
Balloon catheter with sleeve
Cathéter à ballonnette avec gaine

(30) Priorität: 09.06.1990 DE 4018525
(43) Veröffentlichungstag der Anmeldung: 18.12.1991
(73) Patentinhaber: Kaltenbach, Martin, Prof. Dr. med., D-63303 Dreieich (DE)
(72) Erfinder: Kaltenbach, Martin, Prof. Dr. med., D-63303 Dreieich (DE)
(74) Vertreter: Schmitt, Hans, Dipl.-Ing.

(56) Entgegenhaltungen:
- EP-A- 246 998
- DE-A- 3 902 364
- GB-A- 1 547 328
- US-A- 1 690 995
- US-A- 4 723 549
- US-A- 4 796 629

## Beschreibung

Die Erfindung betrifft einen Katheter gemäß dem Oberbegriff des Anspruches 1.

Ein solcher Katheter ist aus der EP-A-246 998 bekannt. Bei dieser bekannten Ausführungsform eines Katheters ist jedoch vorgesehen, die Stützhülse im Bereich der Gefäßverengung zu implantieren. Dabei wird durch die Aufweitung des Ballons auch die Stützhülse aufgeweitet, wobei sie Rastvorrichtungen oder einen Werkstoff aufweist, wodurch die aufgeweitete Position fixiert wird. Nach dem Aufweiten der Stützhülse kann der Ballon wieder entspannt und der Ballonkatheter ohne die Stützhülse entnommen werden, so daß die Gefahr vermindert wird, daß die Gefäßverengung erneut auftritt. Dabei muß in Kauf genommen werden, daß ein beachtlich großer Fremdkörper mehr oder weniger unkontrolliert in dem Gefäß verbleibt. Dabei darf die Stützhülse nur relativ kurz sein, um auch durch Gefäße mit relativ engen Biegungen hindurchgeführt werden zu können.

Aus der US-A-4 796 629 ist ein Ballonkatheter bekannt, bei welchem innerhalb der Ballonhülle harte Stege eingearbeitet sind. Dadurch wird die Ballonwand bereichsweise widerstandsfähiger gemacht und kann in einzelnen Fällen verhärtete Bereiche verdrängen oder zerdrücken. Es bleibt jedoch die Schwierigkeit bestehen, daß zwischen diesen härteren Stegen und Bereichen jeweils Zwischenräume verbleiben müssen, damit der Ballon aus einem geringen Durchmesser heraus aufgeweitet werden kann. Somit ergibt sich nur eine unvollkommene Lösung des Problems der Verdrängung harter Bereiche einer Gefäßverengung. Befindet sich ein solcher harter Bereich beim Einsatz des Katheters genau in dem Zwischenraum zwischen zwei Aussteifungsstegen, kann hier die Ballonwand nachgeben.

Es besteht deshalb die Aufgabe, einen Katheter der eingangs erwähnten Art zu schaffen, mit dem harte Bereiche einer Verengung weitgehend erfaßt und geglättet werden können, ohne daß diese Bereiche abgetragen oder abgefräst werden müssen und ohne daß ein Fremdkörper in dem Gefäßsystem zurückbleiben muß, wobei gleichzeitig eine so biegsame Gestaltung vorgesehen sein soll, daß der Katheter und die Hülse auch bei relativ großer Länge um relativ enge Biegungen von Gefäßen geführt werden können oder sogar in solchen Biegungen zum Einsatz gelangen können.

Diese Aufgabe wird mit den Merkmalen und Maßnahmen des kennzeichnenden Teiles des Patentanspruches 1 gelöst.

Durch den harten, aber federelastischen Werkstoff der Hülse wird erreicht, daß auch härtere oder widerstandsfähigere Bereiche und Vorsprünge einer Verengung von Gefäßen oder Hohlorganen nicht in die weiche Ballonoberfläche eingedrückt werden können, sondern daß solche harten Strukturen mit dem gewünschten Druck des aufweitbaren Bereiches beaufschlagt und dadurch weggedrückt werden. Versuche haben gezeigt, daß durch eine solche aufweitbare Hülse eine Aufweitung und Glättung auch im Bereich harter Gewebebezirke erreichbar ist. Aufgrund der Unnachgiebigkeit der biegsamen Hülse gegen Eindellungen werden sogar die Dehnungskräfte des aufweitbaren Bereiches bei partiell vorstehenden harten Bereichen bevorzugt auf diese übertragen. Durch die Elastizität des federelastischen Werkstoffes der Hülse ergibt sich, daß beim Zurücknehmen des Aufweit-Druckes, insbesondere beim Ablassen des Druckes aus dem Ballon, die Hülse sich wieder selbsttätig auf ihren geringeren Anfangsquerschnitt zurückverformt, so daß der Katheter nach dem Behandeln der Verengung zusammen mit der mit ihm verbundenen Hülse wieder problemlos entnommen werden kann. Es ergibt sich somit die Möglichkeit, einen ganz erheblichen Teil des Umfanges einer Gefäßinnenseite oder eines Hohlorganes zu glätten, ohne daß ein Fremdkörper in dem Gefäß oder Hohlorgan zurückbleiben muß und ohne daß die Gefahr der Ablösung von Teilen der Verengung besteht. Dabei kann vor allem auch ein Ballonkatheter mit einer relativ großen axialen Länge des Ballons und der ihn umschließenden Hülse auch um relativ enge Gefäß-Biegungen herumgeführt und eingeschoben werden, oder umgekehrt kann eine möglichst lange Hülse Verwendung finden, um auch ausgedehnte Stenosen oder einander nah benachbarte Stenosen problemlos und schnell gleichzeitig aufweiten und glätten zu können und dabei vorhandene harte Strukturen erfassen zu können. Obwohl also die Hülse eigentlich sehr widerstandsfähig gegen Verformungen, nämlich gegen Eindellungen sein soll, kann sie aufgrund des oder der Einschnitte, Kerben und dergleichen Schwächungen trotzdem so biegsam gestaltet werden, daß sie auch bei relativ großer Länge um relativ enge Biegungen von Gefäßen geführt oder sogar in solchen Biegungen zum Einsatz gelangen kann.

Um eine gleichmäßige Beaufschlagung nahezu des ganzen oder des gesamten Innenumfanges der Verengung mit der Hülse erreichen zu können, ist es nach einer besonders zweckmäßigen Ausgestaltung der Erfindung vorteilhaft, wenn der in Ausgangsstellung unterhalb einem ihn überlappenden Randbereich der Hülse liegende Randbereich mit dem aufweitbaren Bereich fest verbunden, z.B. verklebt oder verschweißt ist und/oder wenn einer der Randbereiche über wenigstens einen Verbindungssteg mit dem Zuführrohr des Katheters verbunden ist. Es ergibt sich dann, daß die Hülse aufgrund der Überlappung ihrer Randbereiche auch in aufgeweiteter Position noch den gesamten Innenumfang eines Gefäßes oder Hohlorga- nes beaufschlagen kann, sofern das Man der Überlappung in Ausgangsstellung entsprechend groß ist. Durch die sich überlappenden und beim Aufweiten mehr und mehr voneinander gleitenden Randbereiche wird also sichergestellt, daß ständig der gesamte Umfang mit dem harten Hülsenmaterial beaufschlagt bleibt, obwohl eine Aufweitung der Hülse erfolgt und obwohl der Hülsenwerkstoff selbst nicht dehnbar ist. Durch diese Verwendung einer zunächst eingerollten Hülse wird also die Möglichkeit einer Aufweitung durch den Druck des aufweitbaren Bereiches des Katheters, vorzugsweise den Ballondruck geschaffen, ohne daß dabei Bereiche an diesem Ballon gebildet werden, die nicht mit dem harten Material überdeckt sind und an denen sich harte Bereiche der Verengung eindrücken könnten. Durch die Verbindung dieser Hülse mit einem ihrer Randbereiche mit dem Ballon oder dem Zuführrohr des Katheters wird gleichzeitig sichergestellt, daß nach dem Ablassen des Ballondruckes und der daraus resultierenden selbsttätigen Zurückverformung der Hülse diese zusammen mit dem Ballonkatheter wieder aus dem Gefäß entfernt werden kann.

Sowohl das Einführen und Entnehmen des Katheters als auch die Relativbewegung der sich überlappenden Ränder der Hülse werden begünstigt, wenn die Hülse aus gleitfähigem Werkstoff, insbesondere Kunststoff und/oder Metall besteht.

Eine zusätzliche oder abgewandelte Möglichkeit kann darin bestehen, daß die Hülse als schlauchförmiger Kunststoffkatheter aüsgebildet ist, in den der aufweitbare Bereich einführbar ist, und daß der Kunststoffkatheter an der den aufweitbaren Bereich aufnehmenden Stelle etwa in Längsrichtung geschlitzt ist, insbesondere wenigstens zwei oder mehr am Umfang verteilte Längsschlitze und/oder wenigstens einen schraubenlinienförmig angeordneten Schlitz aufweist. Beim Aufweiten werden zwar dann der oder die Schlitze etwas aufgespreizt, jedoch wird nach wie vor der weitaus größte Umfangsbereich einer Gefäßverengung von dem unnachgiebigen, lediglich federelatischen Werkstoff der Hülse beaufschlagt, so daß harte Bereiche gut verdrängt werden können. Gegegenenfalls kann nach einer ersten Aufweitung und anschließenden Reduzierung des Außenumfanges der Kunststoffkatheter etwas verdreht und dann erneut aufgeweitet werden, um die gesamte Innenfläche der Stenose oder Gefäßverengung mit Sicherheit zu erfassen. In jedem Falle ist die Breite der aufklaffenden, eventuell einander gegenüberliegenden Schlitze im Verhältnis zu dem Umfang der harten Hülsenbereiche so gering, daß auch im Schlitzbereich befindliche harte Bereiche, sofern sie eine störende Ausdehnung haben, von den den Schlitzrändern benachbarten Hülsenbereichen erfaßt und zur Seite gedrückt werden.

Um die Rückstellkraft an der Hülse zu verstärken und das Glätten einer Gefäßinnenwand zu verbessern, kann es zweckmäßig sein, wenn die Hülse einen elastischen überzug zum Beispiel aus Latex aufweist. Insbesondere kann dadurch ein übergang an den Rändern der sich überlappenden Bereiche und/oder den Rändern von Schlitzen geschaffen werden.

Das Maß der überlappung der Randbereiche der Hülse an ihren beiden durch das Aufweiten relativ zueinander bewegbaren Längsrändern in Ausgangsstellung kann wenigstens der Größe der Aufweitung des Katheterbereiches, in Umfangsrichtung und der daraus resultierenden Umfangsvergrößerung entsprechen. In diesem Falle kann wiederum der elastische Überzug aus Latex vorteilhaft sein, der dann den Abstand der beiden nebeneinanderliegenden Ränder überbrücken kann.

In vielen Fällen wird jedoch schon eine Aufweitung genügen, bei der die Ränder noch geringfügig überlappend bleiben. Durch die vorerwähnte Maßnahme kann jedoch unter Umständen die Aufweitung soweit fortgesetzt werden, bis die Ränder der Hülse nebeneinanderliegen. Auch eine Fuge kann dabei in Kauf genommen werden, weil der Katheter gegebenenfalls um seine Längsachse gedreht werden kann, also mehrmals innerhalb der Gefäßverengung aufgeweitet, wieder zurückverformt und nach dem Drehen erneut aufgeweitet werden kann. Bei Verengungen, die nur in einem Teil der Zirkumferenz aus hartem Material bestehen, ist der Druck der Hülse auch nur in der betroffenen Region erforderlich.

Die Hülse kann aus einer Folie bestehen und die Länge der Abwickelung der die Hülse bildenden Folie kann wenigstens etwa dem Eineinviertel- bis Eineinhalbfachen, insbesondere etwa dem Doppelten oder Dreifachen des Umfanges des aufweitbaren Bereiches in Ausgangsstellung entsprechen, beziehungsweise kann die Hülse in Ausgangsstellung über wenigstens einen großen Teil ihres Umfanges aus wenigstens zwei Schichten oder Lagen bestehen. Eine entsprechend große Aufweitung der Hülse durch entsprechende Erweiterung des aufweitbaren Endbereiches des Katheters und insbesondere eines Ballons ist möglich.

Es ist möglich, daß die einzelnen Hülsenstücke in Umfangsrichtung gegeneinander verdreht sind, so daß die sich überlappenden Längsränder von Hülsenstück zu Hülsenstück in Umfangsrichtung gegeneinander versetzt sind. Somit entstehen die Reibkräfte bei der Relativbewegung der sich überlappenden Bereiche und Ränder nicht alle an einem Umfangsbereich der Hülse, sondern an verschiedenen Umfangsbereichen, sind also besser verteilt und gegeneinander ausgleichbar. Außerdem wird dadurch die Biegsamkeit der Hülse begünstigt.

Eine abgewandelte oder zusätzliche Ausgestaltungsmöglichkeit besteht darin, daß die Ränder der Hülse schraubenlinienförmig über deren Umfang und Längserstreckung verlaufen. Die sich gegebenenfalls in Ausgangsstellung überlappenden Ränder können also durch eine Schraubenlinienform auch eine gewisse Biegbarkeit der Hülse erlauben und den sich überlappenden Bereich über den Umfang der Hülse und ihre Länge verteilen.

Statt des bei den vorerwähnten Lösungen vorgesehenen Ballons kann auch ein anderes Element als aufweitbarer Bereich am distalen Arbeitsende des Katheters vorgesehen sein, so daß die Hülse auch durch andere Verfahren erweitert werden kann. Außer dem hydraulischen Ballonverfahren ist eine Erweiterund durch mechanische Kräfte möglich. In Betracht kommt beispielsweise die Erweiterung durch Federkraft. Die als aufweitbarer Bereich am distalen Arbeitsende des Katheters dazu verwendbare Feder kann beispielsweise in den in Umfangsrichtung geschlossenen Katheter eingeführt werden und erweitert die Hülse sobald sie aus dem Katheter und seinem distalen Ende herausgeschoben wird, wobei sich die Hülse in axialer Verlängerung dieses die Feder zunächst umschließenden Katheterteiles befindet. Dabei kann eine derartige Feder beispielsweise die Form eines Körbchens oder einer Spindel besitzen, so daß sie auch durch einen englumigen zuführenden Katheter vorgeschoben und für die Rückverformung wieder zurückgezogen werden kann beziehungsweise der Katheter relativ zu der Feder wieder über diese schiebbar ist. Es ist also möglich, daß zum Aufweiten der Hülse gegen ihre Rückstellkraft und/oder die Rückstellkraft des Überzuges eine zunächst zusammengedrückte, insbesondere durch den Führungskatheter oder dergleichen zuführbare Feder, beispielsweise in Form eines Körbchens oder einer Spindel vorgesehen ist, die aus dem sie zusammendrückenden Führungskatheter in das Innere der Hülse vorschiebbar und für die Rückstellung der Hülse wieder zurückziehbar oder durch Vorschieben des Führungskatheters reltiv zu diesem zurückbewegbar ist.

Vor allem bei Kombination einzelner oder mehrerer der vorbeschriebenen Merkmale und Maßnahmen ergibt sich insgesamt ein Katheter, dessen aufweitbarer Bereich oder Ballon eine feste Hülse hat, die durch den innenliegenden Ballon gegen ihre eigene Rückstellkraft und gegebenenfalls zusätzlich die Rückstellkraft eines elastischen Überzuges nach außen gedrückt werden kann, so daß auch im Bereich von eingeengten Gefäßabschnitten befindliche harte Strukturen geglättet und aufgeweitet werden können, ohne daß der weiche Ballon an solchen Strukturen eingedellt und verformt werden kann. Durch die überlappung zweier Längsränder der Hülse in Ausgangsstellung oder die Längsschlitzung der Hülse mit der Aufweitbarkeit der Schlitzbereiche kann die Hülse für das Einführen und Entnehmen des Katheters einen gewünschten kleinen Durchmesser haben und trotzdem auf einen relativ großen Durchmesser aufgeweitet werden. Ein bei extremen Aufweitstellungen oder mit Längsschlitzen versehenen Hülse vorhandener, in Gebrauchsstellung aufklaffender Längsschlitz nimmt nur einen relativ geringen Teil des Gesamtumfanges ein, so daß eine weitgehend gleichmäßige Behandlung des gesamten Umfanges einer Stenose möglich ist oder aber durch mehrmaliges Ansetzen der Hülse nach einem zwischendurch erfolgenden Verdrehen die gleichmäßige Behandlung einer zirkulären Stenose oder Verengung erreicht werden kann.

Nachstehend sind mehrere Ausführungsbeispiele der Erfindung anhand der Zeichnung näher beschrieben.

Es zeigt in zum Teil schematisierter, zum Teil zur Verdeutlichung auch übertriebener Darstellung:
- Fig. 1: einen Längsschnitt durch ein Gefäß mit einer Stenose, in deren Bereich ein erfindungsgemäßer Ballonkatheter mit einer mit ihm verbundenen Hülse eingeführt ist, deren Ränder sich noch überlappen und der noch nicht aufgeweitet ist,
- Fig. 2: eine der Fig. 1 entsprechende Darstellung nach dem Aufweiten des Ballonkatheters und der diesen umschließenden Hülse, wobei zwischen den Hülsenrändern ein Spalt geöffnet ist,
- Fig. 3: eine perspektivische Seitenansicht eines bezüglich seiner Querschnittsabmessung übertrieben groß dargestellten Katheters in Ausgangsstellung,
- Fig. 4: einen Längsschnitt durch einen Katheter mit Ballon und zugehöriger Hülse gemäß Fig. 3 und mit einem die Hülse tragenden Zuführrohr in Ausgangsstellung, jedoch bezüglich des Gesamtquerschnittes zur Verdeutlichung übertrieben groß dargestellt.
- Fig. 5: einen Querschnitt durch den Ballon und die ihn umschließende Hülse gemäß Fig. 4 vor dem Aufweiten,
- Fig. 6: eine der Fig. 4 entsprechende Darstellung nach dem Aufweiten des Ballons und der ihn umschließenden Hülse,
- Fig. 7: einen Querschnitt des aufgeweiteten Ballons mit der ihn umschließenden Hülse analog der Darstellung nach Fig. 5,
- Fig. 8: in schaubildlicher Darstellung eine Hülse aus harter Folie in eingerollter Position,
- Fig. 9: eine der Fig. 8 entsprechende Darstellung, wobei die Hülse von einem Längsrand ausgehend quer und in Umfangsrichtung verlaufende Schlitze, Einkerbungen oder Schwächungen hat, um quer zu ihrer Längsachse biegsam zu sein.
- Fig. 10: eine der Fig. 9 entsprechende Darstellung, bei welcher durch Stege unterbrochene, über einen größeren Umfangsbereich als bei Fig. 9 verlaufende Querschlitze oder Schwächungen zur Erhöhung der Biegsamkeit vorgesehen sind,
- Fig. 11: eine Anordnung, bei welcher die quer zu den sich überlappenden Rändern verlaufenden Schwächungen oder Schlitze zur Erhöhung der Biegsamkeit über einen noch größeren Umfangsbereich als bei der Hülse nach Fig. 10 verlaufen,
- Fig. 12: eine der Fig. 4 entsprechende Darstellung, bei welcher die nicht aufgeweitete Hülse über einen Steg und eine Muffe oder dergleichen mit dem Katheter selbst verbunden ist,
- Fig. 13: eine der Fig. 12 entsprechende Darstellung, bei welcher der Ballon und die Hülse aufgeweitet sind,
- Fig. 14: eine perspektivische Darstellung einer aufgeweiteten Hülse etwa gemäß den Darstellungen gemäß den Figuren 8 bis 11, wobei die sich überlappenden Randbereich schraubenlinienförmig angeordnet sind,
- Fig. 15: die der Fig. 14 entsprechende Hülse mit den sich schraubenlinienförmig überlappenden Längsrändern in Ausgangsstellung,
- Fig. 16: eine Ausführungsform, bei welcher der Ballon und die ihn umschließende Hülse von einem elastischen Überzug umschlossen sind, wobei zur besseren Verdeutlichung zwischen dem Ballon und der Hülse einerseits sowie der Hülse und dem Überzug andererseits ein in Wirklichkeit nicht vorhandener Abstand gezeichnet ist,
- Fig. 17: eine an einem Führungskatheter vorgesehene Hülse mit einem oder mehreren das Aufweiten des Ballons ermöglichenden Längsschlitzen sowie
- Fig. 18: das der Fig. 17 entsprechende Ausführungsbeispiel nach dem Aufweiten der Ballons mit aufklaffendem Längsschlitz.

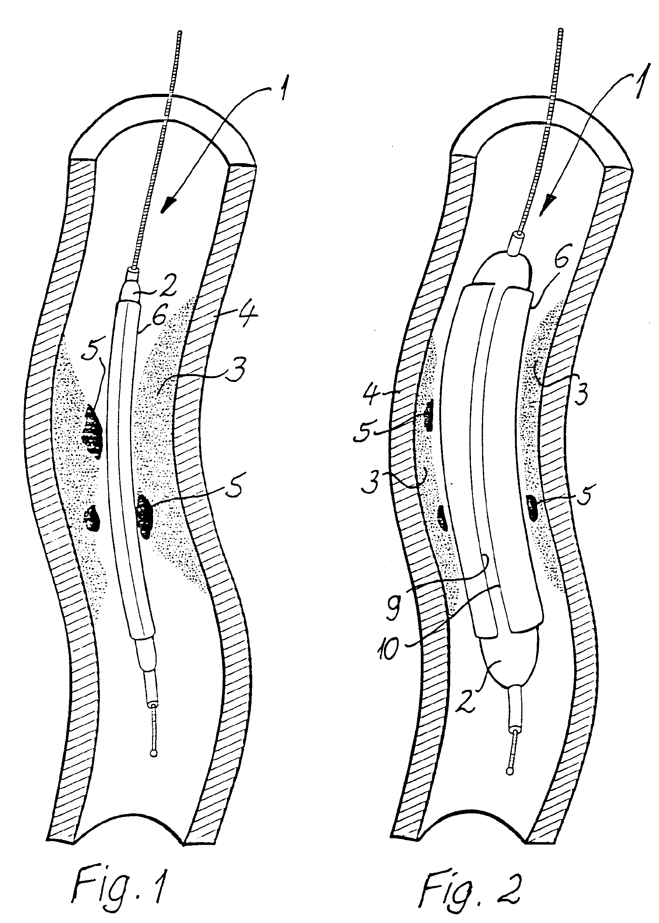

Zunächst sei darauf hingewiesen, daß in den Figuren 1 bis 8 und 12 bis 18 die zur Erlangung der Biegbarkeit der Hülse quer zu ihrer Längserstreckung vorgesehenen, im folgenden noch zu erläuternden Einschnitte, Kerben oder durch Stege verbundenen Hülsenstücke der Übersichtlichkeit wegen nicht dargestellt sind, die in den genannten Figuren dargestellten Ausführungsformen aber nur zusammen mit diesem Merkmal die Erfindung ergeben.

Ein im ganzen mit 1 bezeichneter, vor allem in den Fig. 1 und 2 in Gebrauchsstellung mit seinem Arbeitsende dargestellter Katheter 1 hat an diesem seinem distalen Arbeitsende einen aufweitbaren Ballon 2 zur Behandlung von Stenosen 3 in Gefäßen 4. Dabei ist in Fig. 1 und 2 angedeutet, daß solche Stenosen 3 auch harte Bereiche 5 haben können, die häufig aufgrund ihrer Härte besonders prominent sind bzw. besonders weit in das Innere des Gefäßes 1 vorstehen.

Damit beim Aufweiten des Ballons 2 diese harten Bereiche 5 den Ballon 2 nicht eindellen und unter dem Druck des geweiteten Ballons 2 weggedrückt werden, ist ein großer Teil der Ballonoberfläche mit einer Hülse 6 überdeckt, die Von einer Folie aus hartem, biegsamen Material gebildet ist. Vor allem beim Vergleich der Fig. 1 und 2 erkennt man, daß diese Hülse 6 durch das Füllen und Aufweiten des Ballons 2 an die zu behandelnde Gefäßwand anpressbar ist, weil auch diese Hülse 6 in noch zu beschreibender Weise aufweitbar ist.Aufgrund der Härte ihres Materiales verdrängt sie dabei die gesamte Stenose 3 einschließlich harter Strukturen oder Bereiche 5.

Zumindest in Ausgangsstellung reicht die Hülse 6 gem. Fig. 1, 4, 5 und 8 bis 11 über den gesamten Umfang des Ballons 2 und hat sich in Umfangsrichtung überlappende Ränder oder Randbereiche 7 und 8. Vergleicht man beispielsweise die Fig. 5 und 7, in denen der zunächst nicht gefüllte Ballon (Fig. 5) und dann der aufgeweitete Ballon (Fig. 7) mit einer solchen Hülse 6 dargestellt ist, erkennt man, daß beim Aufweiten des Ballons 2 die sich überlappenden Bereiche 7 und 8 relativ zueinander in Umfangsrichtung verschiebbar sind, so daß also die Hülse 6 in ihrem Durchmesser aufweitbar ist.

Das Maß der Überlappung der Hülse 6 an ihren beiden durch das Füllen und Aufweiten des Ballons 2 relativ zueinander bewegbaren Längsrändern 9 und 10 entspricht in Ausgangsstellung wenigstens der Größe der Aufweitung des Ballons in seiner Umfangsrichtung und der daraus resultierenden Umfangsvergrößerung dieses Ballons 2. Daraus ergibt sich, daß die beiden Ränder 9 und 10 in der extremen Füllungsstellung des Ballons 2 ggf. sogar nebeneinander liegen, wie es in Fig. 2 und 7 angedeutet ist. Bis dahin sind allerdings eventuelle harte Strukturen oder Bereiche 5 einer Stenose 3 bereits derart verdrängt und zur Seite gedrückt, daß ein Eindrücken zwischen den Rändern 9 und 10 ausgeschlossen erscheint. Im Übrigen ist darauf hinzuweisen, daß der in Fig. 7 und 2 angedeutete Abstand der äußeren Rändern 9 und 10 in der extremen Aufweitstellung übertrieben dargestellt ist und eigentlich allenfalls eine schmale Längsfuge entsteht oder in der Mehrzahl der Fälle sogar noch ein gewisser Überlappungsgrad erhalten bleibt, so daß auch in aufgeweitetem Zustand der Ballon 2 von der harten Hülse 6 umschlossen bleibt.

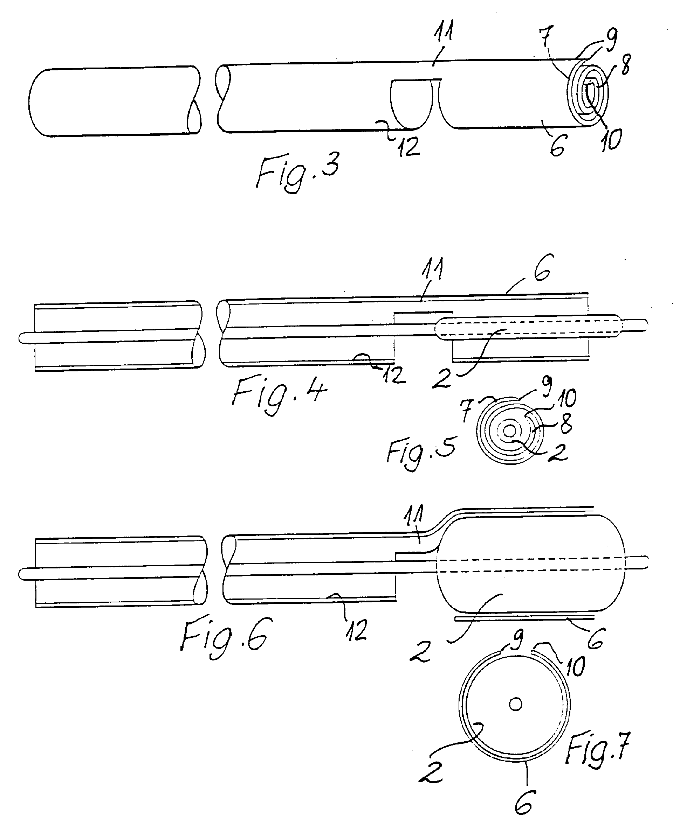

Damit eine kontrollierte Aufweitung der Hülse 6 erfolgt und diese während des Einführens des Katheters 1 ihre Position relativ zu dem Ballon 2 behält, kann der in Ausgangsstellung unterhalb dem äußeren überlappenden Randbereich 7 der Hülse 6 liegende Randbereich 8 mit dem Ballon 2 fest verbunden, zum Beispiel verklebt sein. Im Ausführungsbeispiel ist die Hülse 6 über einen Verbindungssteg 11 mit einem Zuführrohr 12 des Katheters 1 verbunden, wobei der Steg 11 gem. Fig. 3 mit dem außen liegenden Randbereich 7 vorzugsweise einstückig verbunden ist. Es könnte jedoch ebenso vor allem bei einem Zuführrohr 12 mit geringerem Außendurchmesser ein Verbindungssteg 11 zu dem innenliegenden Randbereich 8 vorgesehen sein. Für die relative Gleitbewegung der sich überlappenden Bereiche 7 und 8 ist es günstig, wenn die Hülse aus gleitfähigem Folienwerkstoff, insbesondere einem entsprechend gleitfähigen Kunststoff und/oder Metall besteht. Gleichzeitig ist es vorteilhaft, wenn die Hülse 6 federelastisch ist, also gegen die aus ihrer Federelastizität resultierende Rückstellkraft aufweitbar ist, so daß sie nach dem Ablassen des Druckes aus dem Ballon 2 auch wieder ihre eingerollte und im Außenquerschnitt verminderte Form einnimmt.

Je nach dem, wie groß das Aufweitvermögen des Ballons 2 ist, ist auch eine entsprechend große Überlappung der Bereiche 7 und 8 wünschenswert und zweckmäßig. Die Länge der Abwicklung der die Hülse 6 bildenden Folie kann beispielsweise etwa dem Einfachen, gem. Fig. 5 auch etwa dem Doppelten des Umfanges des Ballons 2 entsprechen bzw. zweifach um diesen Umfang reichen, das heißt es ergibt sich, daß die nicht aufgeweitete Hülse praktisch zweilagig oder mehrlagig ist, während die aufgeweitete Hülse dann nur noch aus einer Folienlage besteht. Es sei an dieser Stelle erwähnt, daß der in Fig. 5 erkennbare Abstand zwischen der Außenseite des Ballons 2 und der Innenseite des inneren Bereiches 8 der besseren Deutlichkeit halber übertrieben dargestellt ist. In Wirklichkeit wird die Hülse 6 und ihre innere Lage 8 unmittelbar an der Außenseite des nicht aufgeweiteten Ballons anliegen, damit dessen Aufweitbewegung sofort auch auf die Hülse aus der harten Folie übertragen wird.

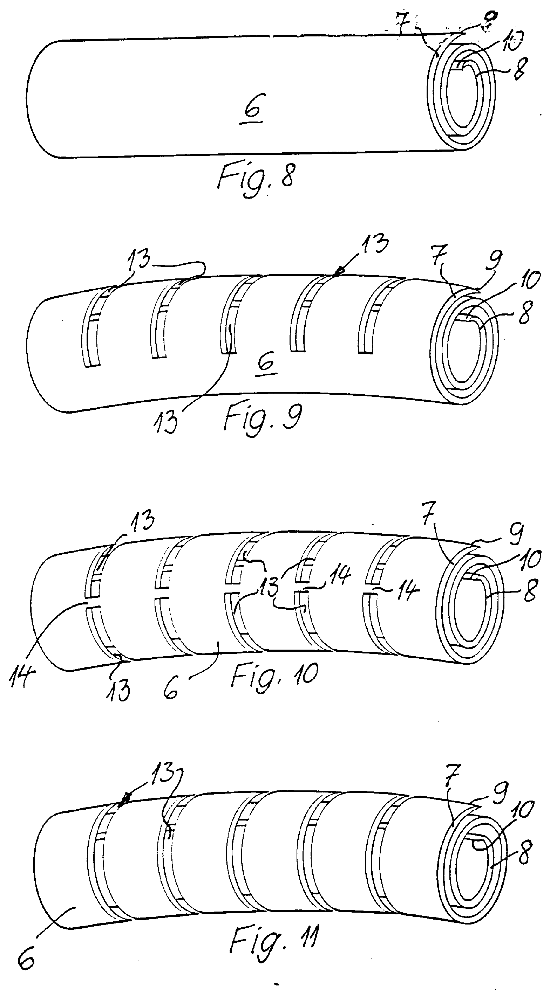

In den Fig. 8 bis 11 sind verschiedene Hülsen 6 im direkten Vergleich zueinander dargestellt. Fig. 8 zeigt dabei eine Hülse, die der der Fig. 1 bis 7 weitgehend entspricht. Bei den weiteren Hülsen 6 ist vorgesehen, daß diese in Umfangsrichtung quer zu ihren sich überlappenden Rändern 9 und 10 verlaufende Einschnitte 13, Kerben oder vergleichbare Schwächungen haben, so daß sie quer zu ihrer Längserstreckung biegsam sind. Vor allem beim Vergleich der Fig. 8 und 9 erkennt man, daß die Hülse nach Fig. 9 aufgrund der Einschnitte 13 etwas biegsam ist. Dies erlaubt eine Vergrößerung der Länge der Hülsen, ohne die Einführbarkeit auch im Bereich enger Krümmungen der Gefäße zu beeinträchtigen.

Fig. 10 zeigt dabei eine Lösung, bei welcher die Hülse 6 durch Stege 14 zwischen den jeweiligen Einschnitten 13 gewissermaßen aus einzelnen Hülsenstücken zusammengesetzt, dabei allerdings einstückig verbunden ist. Es wäre aber auch möglich, einzelne Hülsenstücke durch nachträglich angebrachte Stege 14 zu verbinden.

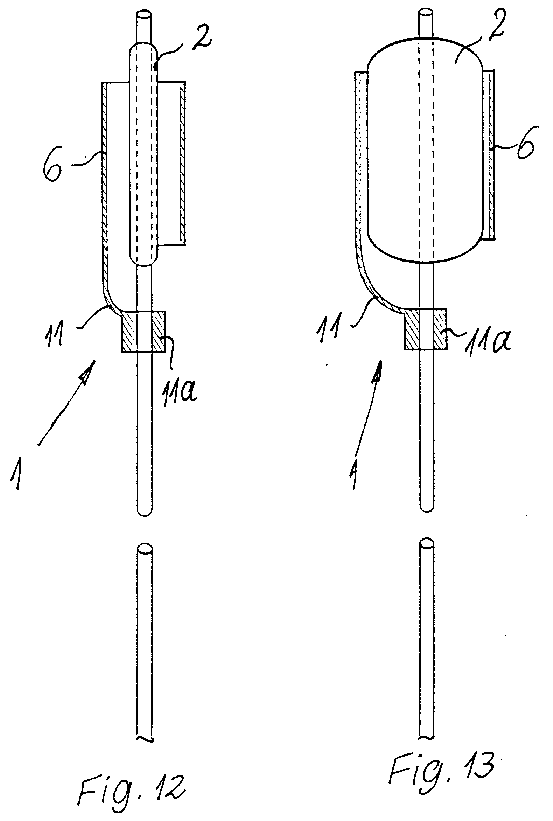

Bei Fig. 11 erkennt man eine über einen großen Umfang verlaufende Schwächung in Form eines Einschnittes 13, wobei unter Umständen Verbindungswege in Umfangsrichtung der Hülse 6 gegeneinander versetzt angeordnet sein könnten.

Wenn die Einschnitte 13 nicht durch die gesamte Foliendicke reichen, bleibt die Hülse auch in Längsrichtung geschlossen, ist aber dennoch in einem gewissen Maße biegsam. Dabei könnten die Einschnitte 13 auch von der Innenseite her in die Hülse eingebracht sein und nicht ganz bis zu ihrer Außenseite reichen, so daß sich eine zwar biegsame, an ihrem Außenumfang aber glatte Hülse ähnlich der der Fig. 8 gäbe.

Wird die erfindungsgemäße Hülse 6 aus ihrer eingerollten Lage gern. Fig.1, 4 und 5 durch Aufweiten des Ballons 2 in eine gespreizte und geweitete Position gem. Fig. 2, 6 und 7 gebracht, können Stenosen und auch harte Bereiche 5 dieser Stenosen gleichmäßig verdrängt und aufgeweitet werden, so daß sich trotz solcher harter Strukturen 5 innerhalb von Stenosen 3 anschließend ein weitgehend gleichmäßig aufgeweiteter Querschnitt innerhalb des Gefäßes 4 ergibt, ohne daß die Ablagerungen der Stenose 3 ganz oder teilweise abgefräßt werden müssen, was das Risiko von Ablösungen von festen Teilchen und auch das Risiko von Verletzungen der Gefäße durch die rotierenden Werkzeuge bedeuten würde. Es werden also die Vorteile des Ballonkatheters bei der Behandlung Von Stenosen 3 ausgenutzt, ohne die Nachteile in Kauf zu nehmen, die durch harte Strukturen 5 und die Eindrückbarkeit der Oberfläche des Ballons 2 entstehen.

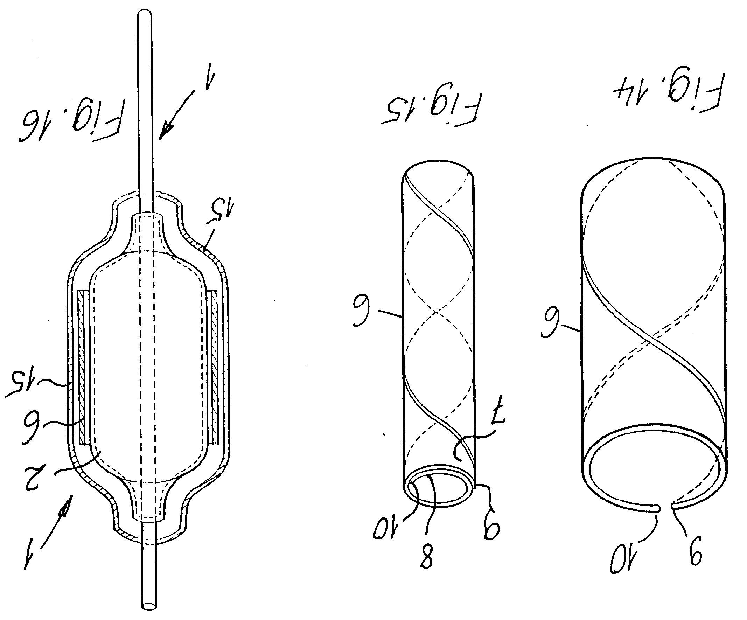

Bei dem Ausführungsbeispiel gemäß den Figuren 12 und 13 ist eine weitgehend übereinstimmende Gestaltung und Funktion wie bei demgemäß den Figuren 1 bis 7 gegeben, wobei jedoch die Hülse 6 statt mit einem Zuführrohr 12 über den Steg 11 und eine Muffe 11a mit dem Katheter 1 selbst verbunden ist.

In Fig. 12 ist der Ballon 2 und somit die Hülse 6 in Ausgangslage und in Fig. 13 in aufgeweiteter Position dargestellt.

Die Figuren 14 und 15 zeigen einerseits in aufgeweiteter und andererseits in Ausgangsstellung die Hülse 6, bei welcher im Gegensatz zu den Ausführungsbeispielen gemäß den Figuren 8 bis 11 die Ränder 9 und 10 schraubenlinienförmig über den Umfang und die Längserstreckung der Hülse 6 verlaufen, so daß die Hülse biegbar und aufweitbar ist. Dabei verdeutlicht Fig. 15, daß sich auch bei diesem Ausführungsbeispiel die schraubenlinienförmig verlaufenden Ränder 9 und 10 in Ausgangslage überlappen können. Es werden somit bei diesem Ausführungsbeispiel die Eigenschaften der Hülse gemäß Fig. 8 und 9 in etwa kombiniert.

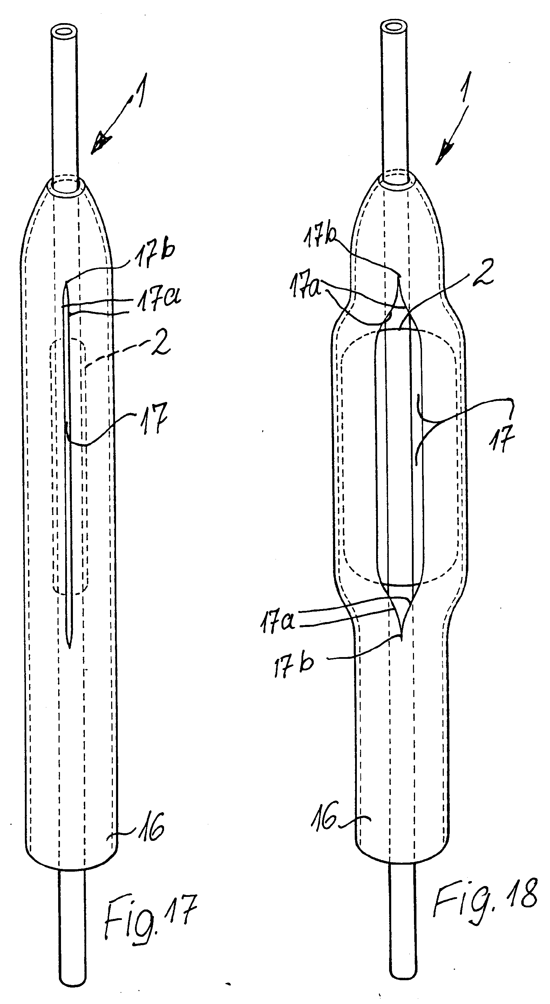

Fig. 16 zeigt eine Lösung, die bei praktisch allen der vorbeschriebenen Ausführungsformen zum Einsatz kommen kann und darin besteht, daß die Hülse 6 einen elastischen Überzug 15 zum Beispiel aus Latex aufweisen kann. Dabei ist auch in Längerstreckungsrichtung dieser Überzug 15 so groß, daß er den gesamten aufweitbaren Bereich beziehungsweise Ballon 2 umfaßt. Die in Fig. 16 erkennbaren Abstände zwischen Ballon 2 und Hülse 6 einerseits sowie Hülse 6 und Überzug 15 andererseits sind nur zur Verdeutlichung vorgesehen und treten in Wirklichkeit nicht auf, daß der aufgeweitete Ballon die Hülse 6 und den Überzug 15 jeweils gegen deren elastische Rückstellkräfte in die in Fig. 16 dargestellte aufgeweitete Postion verformt. Der Überzug 15 verbessert dabei die erwähnten Rückstellkräfte an der Hülse 6, so daß nach dem Zurücknehmen des Druckes im Ballon 2 die Rückverformung auf die zum Zurückziehen das Katheters 1 erforderliche Ausgangsgröße mit umso größerer Sicherheit erfolgt.

Außerdem wird dadurch der gesamte aufweitbare Bereich und die an ihm vorhandenen Übergänge geschmeidiger und glatter gestaltet und eventuelle Ränder oder Kanten der Ränder der Hülse 6 werden weniger spürbar. Dies gilt umso mehr, wenn die Ränder 9 und 10 soweit aufgeweitet werden, daß zwischen ihnen ein Abstand entsteht, der dann weiterhin durch den überzug 15 überbrückt wird.

Die Figuren 17 und 18 zeigen ein Ausführungsbeispiel eines Katheters 1, dem als Hülse ein schlauchförmiger Kunststoffkatheter 16 zugehört, die zu einem erheblichen Teil über den Ballon 2 axial hinausragt. Dieser Kunststoffkatheter 16 ist an der den aufweitbaren Bereich oder Ballon 2 aufnehmenden Stelle etwa in Längsrichtung geschlitzt, wobei zweckmäßigerweise zwei oder mehr am Umfang verteilte Längsschlitze 17 oder gegebenenfalls wenigstens ein schraubenlinienförmig angeordneter Schlitz vorgesehen sein können. Gemäß Fig. 17 kann dabei die Länge des oder der Längsschlitze 17 wenigstens der des aufweitbaren Bereiches oder Ballons 2 entsprechen oder zweckmäßigerweise dessen Länge insbesondere beidseitig um zum Beispiel ein Achtel bis die Hälfte, im Ausführungsbeispiel etwa ein Viertel übertreffen. Dies führt beim Aufweiten des Ballons 2 und dem dadurch bewirkten Aufklaffen des oder der Längsschlitze 17 dazu, daß ein genügend großer Übergang der Schlitzränder 17a zu den Schlitzenden 17b hin ermöglicht wird, wie man es in Fig. 18 deutlich erkennt.

Auch bei diesem Ausführungsbeispiel ist ein elastischer Überzug 15 möglich und zweckmäßig.

Ebenso kann ein solcher elastischer Überzug 1 5 auch dann vorgesehen sein, wenn die Erweiterung des aufweitbaren Bereiches am distalen Arbeitsende des Katheters 1 nicht durch das hydraulische Ballonverfahren, sondern auf mechanischem Wege beispielsweise durch Federkraft erfolgt.

In allen Ausführungsbeispielen weist der Katheter 1 im Bereich seines distalen Arbeitsendes und eines dort vorhandenen aufweitbaren Ballons 2 zur möglichst gleichmäßigen Behandlung von Stenosen 3, die auch harte Bereiche oder Strukturen 5 enthalten können, eine Hülse 6 aus hartem biegsamem Material auf, welche Hülse 6 durch das Aufweiten des Ballons 2 an die Stenose 3 und die Gefäßwand anpreßbar ist, wobei das harte Material der Hülse 6 verhindert, daß der Ballon den harten Strukturen 5 durch Eindellungen ausweicht und die harten Strukturen 5 erhalten bleiben; durch die Verwendung eines federelastischen Werkstoffes für die Hülse 6 und gegebenenfalls durch die Zusätzliche Verwendung eines Überzuges wird dabei erreicht, daß beim Zurückverformen des Ballons 2 auch die Hülse 6 automatisch und selbsttätig wieder in ihre Ausgangslage und zu ihrer Ausgangsgröße zurückverformt wird, so daß sie leicht zusammen mit dem Katheter 1 wieder aus dem Gefäß 4 oder einem Hohlorgan entfernt werden kann.

## Patentansprüche

1. Katheter (1) mit einem an seinem distalen Arbeitsende befindlichen aufweitbaren Bereich, insbesondere Ballon (2), zur Behandlung von Verengungen (3) in Gefäßen (4) oder Hohlorganen, wobei wenigstens ein Teil des aufweitbaren Bereiches mit zumindest einer Hülse (6) aus hartem, biegsamem, elastischem Material überdeckt ist, die über den Umfang des aufweitbaren Bereiches reicht und deren quer zur Umfangsrichtung verlaufende, einander in AusgangsStellung insbesondere berührende oder überlappenae Ränder (9,10) oder Randbereiche (7,8) durch das Aufweiten beim Anpressen an die zu behandelnde Gefäß- oder Organwand in Umfangsrichtung relativ zueinander verschiebbar sind, so daß die Hülse (6) gegen die aus der Elastizität resultierende Rückstellkraft in ihrem Querschnitt aufweitbar ist, **dadurch gekennzeichnet** , daß die Hülse (6) aus federelastischem Werkstoff besteht und bei Verminderung des Umfanges des aufweitbaren Bereiches selbsttätig zurückverformbar ist, daß die Hülse (6) mit dem aufweitbaren Bereich, mit dem Zuführrohr (12) des Katheters oder mit einem den Katheter (1) umschließenden Führungskatheter verbunden ist, daß die Hülse (6) wenigstens einen in Umfangsrichtung und quer zu den Rändern (9,10) oder Randbereichen (7,8) verlaufenden Einschnitt (13), eine Kerbe oder dergleichen Schwächung hat oder aus einzelnen, durch Stege (14) einstückig oder nachträglich verbundenen Hülsenstücken zusammengesetzt ist und daß die Hülse (6) quer zu ihrer Längserstreckung biegbar ist.

2. Katheter nach Anspruch 1, dadurch gekennzeichnet, daß der in Ausgangsstellung unterhalb einem ihn überlappenden Randbereich (7) der Hülse (6) liegende Randbereich (8) mit dem aufweitbaren Bereich fest verbunden, z.B. verklebt oder verschweißt ist und/oder daß einer der Randbe- reiche (7,8) über wenigstens einen Verbindungssteg mit dem Zuführrohr (12) des Katheters (1) verbunden ist.

3. Katheter nach Anspruch 1, dadurch gekennzeichnet, daß die Hülse als schlauchförmiger Kunststoffkatheter (16) ausgebildet ist, in den der aufweitbare Bereich einführbar ist, und daß der Kunststoffkatheter an der den aufweitbaren Bereich aufnehmenden Stelle etwa in Längsrichtung geschlitzt ist, insbesondere wenigstens zwei oder mehr am Umfang verteilte Längsschlitze (17) und/oder wenigstens einen schraubenlinienförmig angeordneten Schlitz aufweist.

4. Kahteter nach einem der Ansprüche 1 bis 3, dadurch gekennzeichnet, daß die Hülse (6) aus gleitfähigem Werkstoff, insbesondere Kunststoff und/oder Metall besteht.

5. Katheter nach einem der Ansprüche 1 bis 4, dadurch gekennzeichnet, daß die Hülse (6) einen elastischen Überzug (15) (z.B.) aus latex aufweist.

6. Katheter nach einem der Ansprüche 1 bis 5, dadurch gekennzeichnet, daß das Maß der Überlappung der Randbereiche (7, 8) der Hülse (6) an ihren beiden durch das Aufweiten relativ zueinander bewegbaren Längsrändern (9,10) in Ausgangsstellung wenigstens der Größe der Aufweitung des Katheterbereiches in Umfangsrichtung und der daraus resultierenden Umfangsvergrößerung entspricht.

7. Katheter nach einem der Ansprüche 1 bis 6, dadurch gekennzeichnet, daß die Hülse aus einer Folie besteht und die Länge der Abwicklung der die Hülse (6) bildenden Folie wenigstens etwa dem Eineinviertel- bis Eineinhalbfachen, insbesondere etwa dem Doppelten oder Dreifachen des Umfanges des aufweitbaren Bereiches in Ausgangsstellung entspricht, beziehungsweise die Hülse (6) in Ausgangsstellung über wenigstens einen großen Teil ihres Umfanges aus wenigstens zwei Schichten oder Lagen bestehz.

8. Katheter nach einem der Ansprüche 1 bis 7, dadurch gekennzeichnet, daß die einzelnen Hülsenstücke in Umfangsrichtung gegeneinander verdreht sind so daß die sich überlappen den Längsränder von Hülsenstück zu Hülsenstück in Umfansrichtung gegeneinander versetzt sind.

9. Katheter nach einem der Ansprüche 1 bis 8, dadurch gekennzeichnet, daß die Ränder (9, 10) der Hülse (6) schraubenlinienförmig über deren Umfang und Längserstreckung verlaufen.

10. Katheter nach einem der vorstehenden Ansprüche, dadurch gekennzeichnet, daß zum Aufweiten der Hülse gegen ihre Rückstellkraft und/oder die Rückstellkraft des Überzuges (15) eine zunächst zusammengedrückte, insbesondere durch den Führungskatheter oder dergleichen zuführbare Feder, beispielsweise in Form eines Körbchens oder einer Spindel, vorgesehen ist, die aus dem sie zusammendrückenden Führungskatheter in das Innere der Hülse (6) vorschiebbar und für die Rückstellung der Hülse (6) wieder zurückziehbar oder durch Vorschieben des Führungskatheter relativ zu diesem zurückbewegbar ist.

## Claims

1. A catheter (1) having at its distal operative end an expansible area, particularly a balloon (2) for treating constrictions (3) in vessels (4) or hollow organs, at least part of the expansible area being covered by at least one sleeve (6) of hard, bendable, elastic material reaching over the circumference of the expansible area, the edges (9, 10) or edge areas (7, 8) of said sleeve - which run transversely of the circumferential direction and in particular contact or overlap each other in the starting position being movable relative to each other in a circumferential direction through the expansion as pressure is exerted against the wall of the vessel or organ to be treated, so that the sleeve (6) is expansible in its cross section against the restoring force resultant from the elasticity, characterized in that the sleeve (6) consists of springy material and is adapted to be restored automatically to its original shape as the circumference of the expansible area reduces, that the sleeve (6) is connected to the expansible area, to the feed tube (12) of the catheter or to a guide catheter surrounding the catheter (1), that the sleeve (6) has at least one slit (13), notch or like reduction running in the circumferential direction and transversely of the edges (9, 10) or edge areas (7, 8) or is composed of individual sleeve pieces integrally connected or subsequently connected by webs (14), and that the sleeve (6) is bendable transversely of its longitudinal expanse.

2. A catheter as claimed in claim 1, characterized in that the edge area (8) which in the starting position lies beneath an overlapping edge area (7) of the sleeve (6) is firmly connected, e.g. adhesively secured or welded, to the expansible area and/or that one of the edge areas (7, 8) is connected to the feed tube (12) of the catheter (1) by way of at least one connecting web.

3. A catheter as claimed in claim 1, characterized in that the sleeve takes the form of a tubular plastic catheter (16), the expansible area being adapted to be introduced therein, and that at the location receiving the expansible area the plastic catheter is slit approximately in a longitudinal direction, in particular has at least two or more longitudinal slits (17) distributed over the circumference and/or at least one slit arranged in the shape of a spiral.

4. A catheter as claimed in any one of claims 1 to 3, characterized in that the sleeve (6) consists of slidable material, particular of plastics material and/or metal.

5. A catheter as claimed in any one of claims 1 to 4, characterized in that the sleeve (6) has an elastic cover (15), for example of latex.

6. A catheter as claimed in any one of claims 1 to 5, characterized in that the extent of overlap of the edge areas (7, 8) of the sleeve (6) at their two longitudinal edges (9,10) movable relative to each other through expansion corresponds in the starting position at least to the amount of expansion of the catheter area in the circumferential direction and to the resulting circumferential enlargement.

7. A catheter as claimed in any one of claims 1 to 6, characterized in that the sleeve consists of a foil and the length of unwinding of the foil composing the sleeve (6) approximates at least one and a quarter to one and a half times, particularly about double or three times the circumference of the expansible area in the starting position, or in the starting position the sleeve (6) consists of at least two layers or plies over at least a large part of its circumference.

8. A catheter as claimed in any one of claims 1 to 7, characterized in that the individual sleeve pieces are circumferentially offset, so that the overlapping longitudinal edges are staggered from sleeve piece to sleeve piece in a circumferential direction.

9. A catheter as claimed in any one of claims 1 to 8, characterized in that the edges (9, 10) of the sleeve (6) run in the shape of a spiral over its circumference and longitudinal expanse.

10. A catheter as claimed in any one of the preceding claims, characterized in that for expanding the sleeve against its restoring force and/or the restoring force of the cover (15), an initially compressed spring is provided, particularly one feedable through the guide catheter or the like and for instance in the shape of a small basket or spindle, said spring being adapted to be advanced from the guide catheter compressing it, into the interior of the sleeve (6), and to be retracted again for restoring the sleeve, or to be moved back relative to the guide catheter by advancing the latter.

## Revendications

1. Cathéter (1) comprenant une zone expansible située à son extrémité distale de travail, en particulier sous forme d'un ballonnet (2), pour le traitement de rétrécissements (3) de vaisseaux (4) ou d'organes creux, sur lequel une partie au moins de la zone expansible est recouverte d'au moins un manchon (6) en matériau dur, flexible et élastique, qui s'étend sur la périphérie de la zone expansible et dont les bords (9, 10) ou les zones marginales (7, 8), orientés transversalement à la direction périphérique et se touchant ou se recouvrant en particulier à la position de repos, sont mobiles l'un par rapport à l'autre dans la direction périphérique par suite de l'expansion lors du pressage contre la paroi du vaisseau ou de l'organe à traiter, de sorte que le manchon (6) est expansible en section droite, à l'encontre de la force de rappel due à l'élasticité, caractérisé en ce que le manchon (6) est fait d'un matériau ayant l'élasticité d'un ressort et est capable de reprendre de lui-même la forme initiale lors de la diminution de la circonférence de la zone expansible, que le manchon (6) est relié à la zone expansible, au tube d'amenée (12) du cathéter ou à un cathéter de guidage qui entoure le cathéter (1), que le manchon (6) présente au moins une incision (13), une entaille ou un affaiblissement analogue orienté dans la direction périphérique et transversalement aux bords (9, 10) ou aux zones marginales (7, 8), ou est constitué de tronçons de manchon reliés d'un seul tenant entre eux ou assemblés par la suite au moyen de barrettes (14), et que le manchon (6) est flexible transversalement à son étendue longitudinale.

2. Cathéter selon la revendication 1, caractérisé en ce que la zone marginale (8) située, à la position de repos, au-dessous d'une zone marginale (7) du manchon (6) qui la recouvre, est reliée de façon fixe à la zone expansible, par exemple par collage ou soudage, et/ou que l'une des zones marginales (7, 8) est reliée par l'intermédiaire d'au moins une barrette de liaison au tube d'amenée (12) du cathéter (1).

3. Cathéter selon la revendication 1, caractérisé en ce que le manchon est réalisé comme un cathéter (16) en matière plastique ayant la forme d'un tuyau et dans lequel peut être introduite la zone expansible, et que le cathéter en matière plastique est fendue, à peu près en direction longitudinale, à l'endroit recevant la zone expansible, où il présente en particulier au moins deux ou davantage de fentes longitudinales (17) réparties sur la périphérie et/ou au moins une fente s'étendant suivant une ligne hélicoïdale.

4. Cathéter selon une des revendications 1 à 3, caractérisé en ce que le manchon (6) est fait d'un matériau glissant, en particulier de matière plastique et/ou de métal.

5. Cathéter selon une des revendications 1 à 4, caractérisé en ce que le manchon (6) comporte une gaine élastique (15), en latex par exemple.

6. Cathéter selon une des revendications 1 à 5, caractérisé en ce que la mesure du recouvrement mutuel des zones marginales (7, 8) du manchon (6), sur ses deux bords longitudinaux (9, 10) déplaçables l'un par rapport à l'autre par l'expansion, correspond au moins, à la position de repos, à la grandeur de l'élargissement de la zone expansible du cathéter dans la direction périphérique et à l'agrandissement de la circonférence qui en résulte.

7. Cathéter selon une des revendications 1 à 6, caractérisé en ce que le manchon est fait d'une feuille et la longueur du développement de la feuille formant le manchon (6) correspond au moins, à peu près, à une fois et un quart jusqu'à une fois et demie, en particulier à peu près au double ou au triple de la circonférence de la zone expansible à la position de repos, ou que le manchon (6) à la position de repos est formé sur au moins une grande partie de sa périphérie d'au moins deux couches ou spires.

8. Cathéter selon une des revendications 1 à 7, caractérisé en ce que les différents tronçons du manchon sont tournés les uns par rapport aux autres dans la direction périphérique, de sorte que les bords longitudinaux, qui se recouvrent, sont mutuellement décalés, dans la direction périphérique, d'un tronçon de manchon à l'autre.

9. Cathéter selon une des revendications 1 à 8, caractérisé en ce que les bords (9, 10) du manchon (6) s'étendent suivant une ligne hélicoïdale sur la périphérie et sur l'étendue longitudinale du manchon.

10. Cathéter selon une des revendications précédentes, caractérisé en ce que, pour expanser le manchon à l'encontre de sa force de rappel et/ou la force de rappel de la gaine (15), on a prévu un ressort, initialement comprimé, par exemple sous la forme d'un petit panier ou d'une vis, pouvant être amené en particulier à travers le cathéter de guidage ou analogue, qui peut être avancé à partir du cathéter de guidage qui le comprime jusqu'à l'intérieur du manchon (6), et qui, en vue du rappel du manchon (6) à la position initiale, peut de nouveau être retiré, ou peut être retiré par rapport au cathéter de guidage par l'avancement de celui-ci.
